Europäisches Patentamt

European Patent Office

Office européen des brevets

(11) Publication number: **0 202 557 B1**

# EUROPEAN PATENT SPECIFICATION

(45) Date of publication of patent specification: **11.09.91**  (51) Int. Cl.⁵: **C07C 209/68**

(21) Application number: **86106349.3**

(22) Date of filing: **09.05.86**

(54) Process for producing monotertiary butyl toluenediamine.

(30) Priority: **17.05.85 US 735426**

(43) Date of publication of application:
**26.11.86 Bulletin 86/48**

(45) Publication of the grant of the patent:
**11.09.91 Bulletin 91/37**

(84) Designated Contracting States:
**AT BE CH DE FR GB IT LI NL SE**

(56) References cited:
**EP-A- 0 082 258**
**EP-A- 0 177 916**
**FR-A- 1 406 739**

(73) Proprietor: **AIR PRODUCTS AND CHEMICALS, INC.**
**P.O. Box 538**
**Allentown, Pennsylvania 18105(US)**

(72) Inventor: **Burgoyne, William Franklin, Jr.**
**1950 Potomac Street**
**Allentown, PA 18103(US)**
Inventor: **Dixon, Dale David**
**Rd. No. 3, Box 337-35**
**Kutztow, PA 19530(US)**

(74) Representative: **Kador & Partner**
**Corneliusstrasse 15**
**W-8000 München 5(DE)**

## Description

This invention pertains to a process for producing monotertiary butyl derivatives of toluenediamine in high selectivity by utilization of an acidic, zeolitic catalyst.

## BACKGROUND OF THE INVENTION

Alkylated aromatic diamines have been known for a substantial period of time and find use in the preparation of polyurethane elastomers. More specifically ortho-alkylated aromatic diamines are widely employed as chain extenders in the urethane process, the aromatic diamine forming a short chain urea linkage to strengthen the resulting elastomer.

Two types of synthesis techniques have generally been used to produce alkylated aromatic diamines, one involving the Friedel-Crafts alkylation of an aromatic hydrocarbon, dinitration of the alkylated aromatic compound and then reduction of the nitro group to the amine. When the alkyl group was tertiary butyl, this technique generally resulted in producing an aromatic amine where the alkyl group is meta to the amine groups. Another technique for alkylating aromatic diamines involved direct alkylation of an aromatic diamine, the alkylation being effected by reacting an aromatic diamine with on olefin or olefin pre-cursor in the presence of either homogeneous or heterogeneous catalytic systems. The resulting product generally contained substantial amounts of ortho-alkylated aromatic diamine, para-alkylated aromatic diamines as well as some N-alkylated aromatic diamine and dialkylated aromatic diamine. In urethane synthesis the ortho-alkylated product has been preferred.

Representative patents which illustrate various catalytic processes for alkylating aromatic amines include:

European patent 0,082,258 shows the styrenation of toluenediamine utilizing a silica-alumina catalyst;

French patent 1,406,739 discloses the alkylation of aniline with propylene in the presence of a zeolite X having a pore size of 13 Angstroms;

British patent 846,226 discloses the preparation of nuclear-butylated phenylamines by reacting aniline with isobutylene in the presence of a neutral bleaching earth of the montmorillonite type; and

British patent 414,574 discloses the reaction of aniline or toluidine with cyclohexene in the presence of Tonsil, a hydrated silica catalyst.

Homogeneous alkylation of aromatic diamines using Friedel-Crafts systems are disclosed in the following patents.

U.S. 2.762,845 discloses the reaction of both aniline and toluidine with ethylene in the presence of mercuric chloride;

U.S. 3,649,692 discloses the reaction of aniline, alpha-napthylamine, and N-alkylated aniline with ethylene in the presence of catalytic systems including mercuric chloride, ethyl aluminum sesquichloride and;

U.S. 3,275,690 which discloses the production of alkylated aromatic amines which include the reaction of toluenediamine with ethylene over a catalyst such as aluminum chloride, aluminum anilide, or mercuric chloride.

Common to the above processes for producing alkylated aromatic amines is the production of a mixture of ortho and para-alkylated diamines and the production of mono and polyalkylated aromatic diamines. As acknowledged, supra, ortho-alkylated aromatic diamines are preferred to the para-alkylated aromatic diamine and in some instances it is preferred to limit alkylation to a single alkyl group.

In european published patent application EP-A-177916, priority date of October 11, 1984, it was disclosed that monotertiary butyl toluenediamine isomers, where the tertiary butyl groups are ortho to the amine group, have excellent utility as chain extenders and are well suited for the preparation of polyurethanes via Reaction Injection Molding (RIM). The 5-tertiary-butyl-2,4-toluenediamine isomer and the 3-tertiary-butyl-2,6- toluenediamine isomer as well as mixtures of the two have utility as a chain extender for the preparation of polyurethanes via a RIM technique. On the other hand, 3,5-ditertiary-butyl-2,6-toluenediamine is more reactive than 3-tertiary-butyl-2,6-toluenediamine for the manufacture of urethanes via RIM techniques and is undesirable from that point of view. It is also less desirable since it is more expensive to produce in view of the fact that an additional molecule of olefin is required to produce the product. Both of the mono tertiary-butyl toluenediamine isomers are effective as antioxidants.

## SUMMARY OF THE INVENTION

This invention pertains to an improved process for producing tertiary butyl toluenediamine isomers, the

2

EP 0 202 557 B1

basic process involving the reaction of 2,4-or 2,6-toluenediamine with isobutylene in the presence of an acidic catalyst under alkylation conditions. The improvement constituting the basis of this invention for selectively producing a monotertiary butyl toluenediamine isomer, particularly the 3-monotertiary-butyl-2,6-toluenediamine isomer without the formation of substantial amounts of 3,5-ditertiary-butyl-2,6-toluenediamine or di-N-tertiary butyl derivatives or N-tert-butyl-ring-tert-butyl-toluenediamine derivatives comprises effecting the reaction between a feedstock containing the 2,4- or 2,6-toluenediamine and isobutylene or precursor thereof in the presence of an acidic zeolite having an effective pore size of from about 6 to about 8 Angstroms (0.6 to 0.8 nm) and a pore configuration such as to admit toluenediamine.

Several advantages are associated with the process of this invention and these advantages include:

an ability to selectively ortho alkylate the 2,6-toluenediamine isomer and selectively limit such alkylation to monoalkylation; e.g., about 10% or less dialkylation;

an ability to produce a monotertiary butyl 2,4 or 2,6-toluenediamine isomer with high conversion levels; e.g., greater than 60% based upon 2,4 or 2,6-toluenediamine;

an ability to form ortho-tert-butyl toluenediamine isomer mixtures which have suitability for use as a chain extender for urethane elastomers; and

an ability to limit alkylation to ring alkylation with a single tert butyl group as opposed to a di-tert butyl derivative where 1 or more butyl groups are N-tert butyl groups.

## DETAILED DESCRIPTION OF THE INVENTION

The feedstock suited for practicing this invention is a feedstock which contains toluenediamine preferably containing a 2,6-toluenediamine isomer. Although the feedstock may consist solely of the 2,6-toluenediamine isomer or the 2,4-toluenediamine isomer it typically will comprise a mixture of the 2,4-toluenediamine isomer and the 2,6-toluenediamine isomer where the 2,6-toluenediamine isomer is present at a level of at least 20% by weight. In most cases a mixture of the 2,4- and the 2,6- toluenediamine isomers in an 80:20 weight ratio is utilized since this weight ratio is typically the reaction product from a toluenediamine plant where the toluenediamine is obtained by the catalytic reduction of dinitrotoluene from the mixed acid nitration of toluene.

With respect to the feedstock, the 2,4-toluenediamine isomer is relatively easy to ortho alkylate at the 5 position while the production of the ring alkylated ditertiary butyl derivative, i.e. 3,5-ditertiary-butyl-2,4-toluenediamine, is rather difficult and more extreme conditions are required to effect this reaction. It is believed the 3,5-ditertiary butyl 2,4-toluenediamine isomer is difficult to achieve primarily because of steric hindrance at the 3-position. Even though the di-ring alkylated derivative of 2,4-toluenediamine is difficult to achieve the N-alkylation can be achieved. The N-alkylated derivatives even though disubstituted, are difficult to separate from the monoalkylated ring t-butyl TDA. Examples are 2-amino-5 tert-4-(tert butylamino) toluene and 2-(tert butylamino)-5 tert butyl-4 aminotoluene. The 2,6-toluenediamine isomer in contrast to 2,4-toluenediamine is relatively easy to alkylate with an olefin or olefin precursor such as isobutylene, and butyl substituents can attach at the 3 and 5 positions.

There are essentially two reasons the 3,5-ditertiary butyl-2,6-toluenediamine isomer is undesired. One is that it is more reactive than the monoalkylated 3-tertiarybutyl-2,6-toluenediamine as a chain extender for RIM processing. Secondly, the disubstituted product requires two moles of olefin for each mole of product, further decreasing the cost-effectiveness of this product relative to the mono-tert-butylated product. There are equally good reasons why the disubstituted N-alkylate derivatives of toluerediamine such as the N-(tert butylamino)-tert-butyl-aminotoluene derivatives of the 2,4- and 2,6-toluenediamine isomers are not desired; one is that the disubstituted N-alkylate derivatives are difficult to separate from the mono ring butyl tert derivative and two like the di-ring alkylated derivatives, two moles of olefin are utilized.

This process which allows production of predominantly mono-ring alkylated product also allows production of predominantly mono-N-alkylated product and largely eliminates formation of di-N-tertiary-butyl toluenediamine. Less selective catalysts produce disubstituted N-alkylate which are difficult, if not impossible, to separate by distillation from the desired mono-ring alkylated products. Thus 5-tertiary-butyl-2,4-toluenediamine and 3-tertiary-butyl-2,6-toluenediamine may be obtained in pure form by distillation when alkylation is controlled to afford a mono-substituted product stream. By mono substituted it is meant there is only one ring alkyl group or one N-alkylated groups.

Ring alkylation of the 2,4- and the 2,6-toluenediamine is effected by reacting the isomer with isobutylene or isobutylene precursor such as tert-butanol. In the case of tert-butanol or other isobutylene precursor, it is believed that the isobutylene molecule is formed in situ; e.g., on dehydration of the tert-butanol. Reaction conditions for carrying out ring alkylation include temperatures from about 100 to about 250°C; preferably, 160-200°C and pressures from about 15 to 2,000 psig (4.46 to 138.9 bar) and

3

preferably from about 100 to 1,000 psig (7.9 to 70 bar). Typically the pressure or the temperature is controlled within the range specified to optimize selectivity and conversion to the desired product. Isobutylene will be charged to the reaction zone to provide a molar ratio of about 1 to 10 moles isobutylene per mole of toluenediamine.

The key to selectively producing the ortho butylated toluenediamine isomers, i.e., 5-tertiary-butyl-2,4-toluenediamine and 3-tertiary-butyl-2,6-toluenediamine with less than about 5% by weight ditertiary-butyl-toluenediamine isomer, and obtaining high conversion e.g., converting greater than 60% by weight of feedstock toluenediamine is in the utilization of a zeolite catalyst. The zeolite is further characterized in that the effective pore size of the zeolite is from about 6 to about 8 Angstroms (0.6 to 0.8 nm) and a pore configuration within the same range to admit toluenediamine molecules. When the pore size of the zeolite is less than about 6 Angstroms (0.6 nm) the zeolite appears to be relatively ineffective for producing the mono ortho-tert-butylated toluenediamine isomer at conventional reaction temperatures and when the effective pore size is larger than about 8 Angstroms (0.8 nm) more ditertiary butyl toluenediamine isomer is produced.

Crystalline zeolites are well-known and basically are alumino-silicates where the mole ratio of silica to alumina is very high e.g., greater than 3:1. These zeolites may be substituted with various cations to alter acidity and thus their activity and these cations may also alter the effective pore sizes and thus further alter their catalytic activity. Two common types of zeolites are the X zeolite and the Y zeolite with the Y zeolite typically having a higher silica content. Typically a Y zeolite will have a silica to alumina molar ratio of about 3:1. Others include the ZSM family, faujasite, erionite, zeolite A and so forth. Y, because of its silica to alumina ratio and pore configuration, is preferred.

As noted, the acidity of zeolites can be controlled by utilizing various cations in place of the sodium atom in a synthetic zeolite. The preferred cations suited for the reaction include the rare earth metals such as lanthanum, cerium, praseodymium, as well as various transition metals, and hydrogen. Basic metal exchanged zeolites, e.g. sodium exchanged zeolites are relatively inactive. For this reason the highly or strongly acidic zeolites, i.e. hydrogen, rare earth metal exchanged or those with high silica to alumina ratios are preferred.

The pore size of zeolites may be altered to either larger or smaller sizes by a variety of known means. For example, it is known that pore sizes may be reduced by chemical modification e.g. addition of oxides of boron and phosphorus, or coke formation. It is therefore possible that a pore size of greater than 8 Angstroms (0.8 nm) may be reduced through modification to yield a zeolite having an effective pore size of 8 Angsroms (0.8 nm) and utilized in the process. Conversely, the zeolite pore size may be increased. The primary method of enlarging pore size is through removing alumina from the catalyst structure. The main methods for removing alumina are hydrothermal treatment (steaming) and acid washing. Thus, a zeolite with a pore size of less than 6 Angstroms (0.6 nm) may be expanded to have an effective pore size of greater than 6 Angstroms (0.6 nm) and utilized in the process. Dealumination can also increase acidity of the zeolite.

The following examples are provided to illustrate various embodiments of the invention and are not intended to restrict the scope thereof. These embodiments illustrate the butylation of toluenediamine isomers and mixtures of these isomers.

Example 1

Alkylation of a 80:20 Mixture of the 2,4- and 2,6-isomers of toluenediamine with Isobutylene over H-Y zeolite

A 15.00 g. portion of H-Y zeolite (powder) having a pore size of 7.4 Angstroms (0.74 nm) 120.0 g. (0.98 mol) of 2,4-toluenediamine, and 30.0 g. (0.25 mol) of 2,6-toluenediamine were charged to a 1000 cc Hastalloy C pressure vessel equipped with a mechanical stirrer. The vessel was sealed and purged with nitrogen, leaving a 217 psig (16 bar) nitrogen blanket. The contents were heated to 180°C with stirring. Isobutylene (280 g., 4.98 mol) was then added over 15 minutes, resulting in an initial reaction pressure of 1271 psig. (88.7 bar) The reaction mixture was maintained at 180°C for 18 hours with constant stirring and then cooled to 150°C. Stirring was discontinued at this time and the residual pressure was vented. Upon removal of the catalyst by hot filtration, a product mixture of the following composition was obtained:

|  | Mole% |
|---|---|
| 2,4-toluenediamine | 19.09 |
| 2,6-toluenediamine | 6.30 |
| 2-(tert-butylamino)-4-aminotoluene | 2.03 |
| 2-amino-4-(tert-butylamino)toluene | 8.11 |
| 5-tert-butyl-2,4-toluenediamine | 48.79 |
| 3-tert-butyl-2,6-toluenediamine | 12.73 |
| 2-(tert-butylamino)-5 tert-butyl-4-aminotoluene | 1.60 |
| 2-amino-5-tert-butyl-4-(tert-butylamino)toluene | 0.55 |
| 2-(tert-butylamino)-5-tert-butyl-6-aminotoluene | trace |
| 3,5-di-tert-butyl-2,6-toluenediamine | 0.81 |
|  | 100.00% |

These results show that H-Y zeolite was extremely effective in producing a mono tert-butylated toluenediamine. There was a minor amount of N-butylated toluenediamine produced but this product is suited for recycle and conversion to ring alkylated product. Only a small percent, e.g., about 3% of ditertiary butyltoluenediamine products (including ring and N-alkylated) were produced while conversion was about 70%.

Example 2

Preparation of 5-tert-butyl-2,4-toluenediamine

A 15.0 g. portion of powdered H-Y zeolite having a pore size of about 7.4 Angstroms (0.74 nm) and 150.0 g. (1.23 mol) of 2,4-toluenediamine were charged to a 1000 cm$^3$ Hastalloy C pressure vessel equipped with a mechanical stirrer. The vessel was sealed and purged with nitrogen leaving a residual 225 psig (16.5 bar) nitrogen blanket. The vessel contents were heated to 180°C with stirring at 500 rpm. Isobutylene (279.0 g., 4.98 mol) was then added over 2 hours, resulting in 1225 psig (85.5 bar) vessel pressure. This provided a mole ratio of 4.05 isobutylene to 1 mole toluenediamine. The reaction mixture was maintained at 180°C for 16 hours with constant stirring. The contents were cooled to 150°C and then stirring was discontinued and the residual pressure vented. Removal of the catalyst by hot filtration afforded the following product mixture:

| | Mole% |
|---|---|
| 2,4-toluenediamine | 15.59 |
| 2-(tert-butylamino)-4-aminotoluene | 1.66 |
| 2-amino-4-(tert-butylamino)toluene | 8.02 |
| 5-tert-butyl-2,4-toluenediamine | 71.60 |
| 2,4-di(tert-butylamino)toluene | 0.20 |
| 2-(tert-butylamino)-5-tert-butyl-4-amino-toluene | 1.38 |
| 2-amino-5-tert-butyl-4-(tert-butylamino)toluene | 0.55 |
| | 99.00% |

The above results show that H-Y zeolite is effective for producing an orthobutylated toluene diamine with high selectivity to the monoisomer and modest selectivity to the N-tert-butylaminotoluenediamine derivatives. Lesser quantities of di-tert-butylated products can be produced by operating at slightly lower temperature and thus at slightly lower conversion. Even so conversion was above 80% and less than 3% ditertiary product was produced.

Example 3

Preparation of 3-tert-butyl-2,6-toluenediamine

A 15.0 g. portion of powdered H-Y zeolitote catalyst and 140.0 g. (1.15 mol) of 2,6-toluenediamine were charged to a 1000 cm³ Hastalloy C pressure vessel equipped with a mechanical stirrer as was done in Example 2. The vessel was sealed and purged with nitrogen leaving a residual 200 psig (14.8 bar) nitrogen blanket at room temperature. The contents were heated to 180°C with stirring. Isobutylene (267 g., 4.76 mol) was then added to the reaction mixture over 20 minutes, resulting in an initial reaction pressure of 1100 psig. (76.9 bar) This provided a molar ratio of 4.1:1 isobutylene to toluenediamine. The reaction mixture was maintained at 180°C for 39 hours with constant stirring. The reaction product was isolated by the procedure used in Example 2 and consisted of the following composition:

| | Mole % |
|---|---|
| 2,6-toluenediamine | 30.48 |
| 2-(tert-butylamino)-6-aminotoluene | 9.79 |
| 3-tert-butyl-2,6-toluenediamine | 56.13 |
| 2-(tert-butylamino)-5-tert-butyl-6-aminotoluene | 1.19 |
| 3,5-di-tert-butyl-2,6-toluenediamine | 1.28 |
| | 98.87% |

The results in terms of conversion and selectivity were similar to those obtained for the conversion of the 2,4-isomer in Example 2. A lesser quantity of di-tert-butylated product can be produced at slightly lower conversion. Conversion was in excess of 70% and selectivity to ditertiary butyl isomers less than 4%.

Example 4

Comparative Example

A 150.0 g. portion of an amorphous silica-alumina catalyst consisting of 13% alumina and 87% silica and 1500 g. (12.28 mol) of 2,6-toluenediamine were charged to a 1 gallon (3.785 dm$^3$) stainless steel pressure vessel equipped with a mechanical stirrer. The vessel was sealed and purged with nitrogen, leaving a 16 psig (2.1 bar) nitrogen blanket. The contents were heated to 200°C with stirring. Isobutylene (870 g., 15.5 mol) was then added over 30 minutes, resulting in an initial reaction pressure of 970 psig. (67.9 bar) This provided a mole ratio of 1.26 1 isobutylene to toluenediamine which was well below that employed in Example 3. The reaction mixture was maintained at 200°C for 45 hours with constant stirring. The reaction product was isolated by the procedure used in Example 3 and consisted of the following composition:

|  | Mole % |
|---|---|
| 2,6-toluenediamine | 43.34 |
| 2-(tert-butylamino)-6-aminotoluene | 3.30 |
| 3-tert-butyl-2,6-toluenediamine | 42.36 |
| 2-(tert-butylamino)-5-tert-butyl-6-aminotoluene | 1.82 |
| 3,5-di-tert-butyl-2,6-toluenediamine | 8.60 |
|  | 99.42% |

The results show that conversion was much lower using the silicaalumina catalyst as compared to the use of H-Y zeolite in Example 3 even though the run was conducted at higher temperature and for a longer time. The results also show greater conversion to the di-tertiary butyltoluenediamine, i.e., about 10% at 55% total conversion, whereas the Example 3 product contained less than 4% by weight dibutylated product at greater than 70% total conversion. And, the greater levels of di-tertiary butyltoluenediamine were obtained at lower isobutylene to toluenediamine mole ratios than were used with the H-Y zeolite in Example 3.

Example 5

Comparative Example - Synthesis of 5-tert-butyl-2,4-toluenediamine

The procedure of Example 2 was followed except for the utilization of a different catalyst and the utilization of a 300 cm$^3$ Hastalloy C pressure vessel equipped with a mechanical stirrer. Approximately 100 grams (0.819 moles) of 2,4-toluenediamine were charged to the vessel along with 5 grams of 36% aqueous hydrochloric acid. The vessel was sealed and purged with nitrogen, leaving a 33 psig (3.3 bar) nitrogen blanket. The vessel contents then were heated to 180°C with continuous stirring. Isobutylene then was introduced into the reactor and 53.4 grams (0.96 moles) was added over 15 minutes. On addition of the isobutylene, the pressure in the reactor increased to 766 psig. (53.8 bar) This feed mixture provided a mole ratio of 1.17 moles isobutylere to toluenediamine. The reaction mixture was maintained at 180°C for 24 hours with constant stirring. At the end of the 24 hour period the pressure had dropped to 524 psig. (37.2 bar) The contents were then cooled to 160°C and stirring discontinued. At that time the reactor was vented and the reaction product analyzed for composition by gas chromotography.

|  | Mole Percent |
| --- | --- |
| 2,4-toluenediamine | 50.70 |
| 2-(tert-butylamino)-4-aminotoluene | 1.84 |
| 2-amino-4-(tert-butylamino)toluene | 12.71 |
| 5-tert-butyl-2,4-toluenediamine | 26.71 |
| 2,4-di(tert-butylamino) toluene | 1.31 |
| 2-(tert-butylamino)-5-tert-butyl-4-aminotoluene | 5.28 |
| 2-amino-5-tert-butyl-4-(tert-butylamino) toluene | 1.45 |
|  | 100.00% |

These data show conversion of toluenediamine to ring-alkylated product was much lower than was obtained with H-Y zeolite in Examples 1, 2 and 3, e.g., about 50% compared to about 85% for the zeolite. Even though conversion was much lower, selectivity was also less to monotertiary butyl toluenediamine. Approximately 8% of the product obtained was the undesirable di-tert-butylated product as compared to less than 4% in Examples 2 and 3 and less than 3% in Example l.

Example 6

Comparative Example

The procedure of Example 5 was followed to produce tertiary butyltoluenediamine by using 15 grams of powdered montmorillonite clay in place of the hydrochloric acid catalyst. As in Example 5, the reaction vessel was purged with nitrogen and then the contents were heated to 180°C with stirring. Approximately 278 grams or 4.95 moles of isobutylene were then added to the reaction mixture over 20 minutes. This provided a mole ratio of 4.0 moles isobutylene to toluenediamine. The initial reaction pressure increased to 1210 psig (84.5 bar) and the contents were maintained at 180°C for 23 hours. At that time the contents were cooled to 150°C and the reactor vented. The catalyst then was removed by hot filtration.

The reaction product was analyzed and contained the following:

|  | Mole % |
| --- | --- |
| 2,4-toluenediamine | 57.82 |
| 2-(tert-butylamino)-4-aminotoluene | 5.49 |
| 2-amino-4-(tert-butylamino)toluene | 18.27 |
| 5-tert-butyl-2,4-toluenediamine | 16.85 |
| 2,4-di(tert-butylamino) toluene | 0.42 |
| 2-(tert-butylamino)-5-tert-butyl-4-aminotoluene | 0.47 |
| 2-amino-5-tert-butyl-4-(tert-butylamino) toluene | 0.27 |
|  | 99.59% |

8

The above data show that overall conversion was much lower than reported in Examples 1, 2 and 3, i.e., less than 50%, and even at such low conversion there was some di-tert-butylated product. The basic problem here is that even though conversion was low it was not selective to ring alkylated product. Over half of the product was to N-alkylated material whereas in Examples 1, 2 and 3 better than 70% of the aromatic amine was converted to ring alkylated product.

Example 7

Comparative Example

A 15.0 g. portion of an amorphous catalysts consisting of 13% alumina and 87% silica, 120 g. (0.98 mol) of 2,4-toluenediamine, and 30 g. (0.25 mol) of 2,6-toluenediamine (80:20) were charged to a 1000 cm$^3$ Hastalloy C pressure vessel equipped with a mechanical stirrer as was done in Example 1. The vessel was sealed and purged with nitrogen, having a 214 psig (15.5 bar) nitrogen blanket. The contents were heated to 200°C with stirring. Isobutylene (280 g.. 4.98 mol) was then added over 15 minutes, resulting in an initial reaction pressure of 1565 psig. (108.9 bar) The reaction mixture was maintained at 200°C for 20 hours with constan stirring. The contents were cooled to 150°C, stirring was discontinued and the residual pressure was vented. Removal of the catalyst by hot filtration afforded the following product mixture.

| | Mole% |
|---|---|
| 2,4-toluenediamine | 32.71 |
| 2,6-toluenediamine | 8.51 |
| 2-(tert-butylamino)-4-aminotoluene | 1.88 |
| 2-amino-4-(tert-butylamino)toluene | 7.84 |
| 2-(tert-butylamino)-6-aminotoluene | 0.76 |
| 5-tert-butyl-2,4-toluenediamine | 33.85 |
| 3-tert-butyl-2,6-toluenediamine | 9.36 |

| | Mole% |
|---|---|
| 2-(tert-butylamino)-5-tert-butyl-4-aminotoluene | 2.25 |
| 2-amino-5-tert-butyl-4-(tert-butyl-amino)toluene | 0.53 |
| 2-amino-5-tert-butyl-4-(tert-butyl-aminotoluene | 0.54 |
| 3,5-di-tert-butyl-2,6-toluenediamine | 1.77 |
| | 100% |

This example can be compared directly with Example 1 and from these data it can be seen that the level of di-tert-butyl-2,6-toluenediamine isomer is much higher with silica-alumina than with a zeolite of the type used in Example 1. The data also show a lower conversion of 2,4-toluenediamine to the ring alkylated product than was obtained with the H-Y zeolite even though the reaction temperature was 20 degrees C higher.

**Claims**

1. In a process for reacting an aromatic amine with an olefin or olefin precursor to form a ring alkylated aromatic amine, said reaction being carried out in the presence of an acidic catalyst, the improvement for selectively forming a monotertiary butyl toluenediamine at high conversions which comprises:
   reacting a toluenediamine feedstock with isobutylene or isobutylene precursor; and
   effecting said reaction in the presence of an acidic zeolite catalyst, said zeolite having an effective pore size from about 6 to 8 Angstroms (0.6 to 0.8 nm).

2. The process of Claim 1 wherein said reaction is carried out at a temperature of from 150 to 225°C and a press fire of from 50 to 2000 psig. (4.46 to 138.9 bar).

3. The process of Claim 2 wherein said zeolite is exchanged with a hydrogen ion or a rare earth metal ion.

4. The process of Claim 3 wherein said zeolite is a Y zeolite.

5. The process of Claim 1 wherein said feedstock contains 2,6-toluenediamine.

6. The process of Claim 4 wherein the toluenediamine feedstock contains approximately 20% by weight of the 2,6-toluenediamine isomer and the balance consists essentially of the 2,4-toluenediamine isomer.

7. The process of Claim 6 where the reaction between toluenediamine and isobutylene is effected within a temperature range from 160-200°C.

8. The process of Claim 7 wherein said feedstock is 2,6-toluenediamine.

9. The process of Claim 8 wherein said feedstock is 2,4-toluenediamine.

10. The process of Claim 3 wherein said zeolite is an X zeolite.

**Revendications**

1. Dans un procédé de réaction d'une amine aromatique avec une oléfine ou un précurseur d'oléfine pour former une amine aromatique alcoylée sur le cycle, ladite réaction étant effectuée en présence d'un catalyseur acide, le perfectionnement pour la formation sélective d'une mono-tert-butyl-toluènediamine avec des conversions élevées qui consiste à :
   faire réagir une matière première de type toluènediamine avec de l'isobutylène ou un précurseur de l'isobutylène ; et
   effectuer ladite réaction en présence d'une zéolite acide catalytique, ladite zéolite ayant une taille effective des pores d'environ 6 à 8 Å (0,6 à 0,8 nm).

2. Le procédé de la revendication 1, dans lequel ladite réaction est effectuée à une température de 150 à 225°C et à une pression de 50 à 2 000 psig (4,46 à 138,9 bars).

3. Le procédé de la revendication 2, dans lequel ladite zéolite est échangée avec un ion hydrogène ou un ion de métal des terres rares.

4. Le procédé de la revendication 3, dans lequel ladite zéolite est une zéolite Y.

5. Le procédé de la revendication 1, dans lequel ladite matière première contient de la 2,6-toluènediamine.

6. Le procédé de la revendication 4, dans lequel la matière première constituée de toluènediamine contient environ 20 % en poids de l'isomère 2,6-toluènediamine, le reste étant constitué essentiellement de l'isomère 2,4-toluènediamine.

7. Le procédé de la revendication 6, dans lequel la réaction entre la toluènediamine et l'isobutylène est effectuée dans une gamme des températures de 160 à 200° C.

8. Le procédé de la revendication 7, dans lequel ladite matière première est la 2,6-toluènediamine.

9. Le procédé de la revendication 8, dans lequel ladite matière première est la 2,4-toluènediamine.

10. Le procédé de la revendication 3, dans lequel ladite zéolite est une zéolite X.

**Patentansprüche**

1. Bei einem Verfahren zur Reaktion eines aromatischen Amins mit einem Olefin oder einer Olefin-Vorstufe zur Bildung eines kernalkylierten aromatischen Amins, wobei diese Reaktion in Gegenwart eines sauren Katalysators durchgeführt wird, umfaßt die Verbesserung zur selektiven Bildung des Mono-tert.-butyl-toluoldiamins mit hoher Umwandlung:
   die Reaktion des Toluoldiamin-Ausgangsmaterials mit Isobutylen oder einer Isobutylen-Vorstufe und
   die Durchführung der Reaktion in Gegenwart eines sauren Zeolithkatalysators, wobei dieser Zeolith eine effektive Porengröße von etwa 6 bis 8 Angström (0,6 bis 0,8 nm) aufweist.

2. Verfahren nach Anspruch 1, worin die Reaktion bei einer Temperatur von 150 bis 225° C und einem Druck von 50 bis 2000 psig (4,46 bis 138,9 bar) durchgeführt wird.

3. Verfahren nach Anspruch 2, worin der Zeolith mit einem Wasserstoffion oder einem Metallion der Seltenen Erden ausgetauscht ist.

4. Verfahren nach Anspruch 3, worin der Zeolith Zeolith Y ist.

5. Verfahren nach Anspruch 1, worin das Ausgangsmaterial 2, 6-Toluoldiamin enthält.

6. Verfahren nach Anspruch 4, worin das Toluoldiamin-Ausgangsmaterial etwa 20 Gew.-% des 2,6-Toluoldiamin-Isomers enthält und der Rest im wesentlichen aus dem 2,4-Toluoldiamin-Isomer besteht.

7. Verfahren nach Anspruch 6, worin die Reaktion zwischen Toluoldiamin und Isobutylen in einem Temperaturbereich von 160-220° C durchgeführt wird.

8. Verfahren nach Anspruch 7, worin das Ausgangsmaterial 2,6-Toluoldiamin ist.

9. Verfahren nach Anspruch 8, worin das Ausgangsmaterial 2,4-Toluoldiamin ist.

10. Verfahren nach Anspruch 3, worin der Zeolith Zeolith X ist.